# EUROPEAN PATENT SPECIFICATION

(11) **EP 4 312 731 B1**
(45) Date of publication and mention of the grant of the patent: **02.09.2026**
(21) Application number: 22876797.6
(22) Date of filing: 27.09.2022
(51) Int. Cl.: A61B 5/00, A61B 5/103, G06T 7/00, G06T 3/40, G06T 7/90, G06T 7/11, G06N 3/08, G06N 3/02

(54) **ELECTRONIC DEVICE FOR DETERMINING SKIN INFORMATION USING HYPER SPECTRAL RECONSTRUCTION**
ELEKTRONISCHE VORRICHTUNG ZUR BESTIMMUNG VON HAUTINFORMATIONEN MITTELS HYPERSPEKTRALREKONSTRUKTION
DISPOSITIF ÉLECTRONIQUE POUR DÉTERMINER DES INFORMATIONS CUTANÉES À L'AIDE D'UNE RECONSTRUCTION HYPER SPECTRALE

(30) Priority: 29.09.2021 IN 202141044300; 12.08.2022 IN 202141044300
(43) Date of publication of application: 07.02.2024
(73) Proprietor: Samsung Electronics Co., Ltd., Suwon-si, Gyeonggi-do 16677 (KR)
(72) Inventor: GOYAL, Ashish, Bengaluru Karnataka 560037 (IN); TIWARI, Vijay Narayan, Bengaluru Karnataka 560037 (IN)
(74) Representative: Appleyard Lees IP LLP
(86) International application number: PCT/KR2022/014431
(87) International publication number: WO 2023/055025

(56) References cited:
- EP-A1- 3 741 290
- WO-A1-2020/146489
- US-A1- 2007 016 079
- US-A1- 2015 003 713
- US-A1- 2017 150 903
- US-B2- 9 117 133
- SHARMA NEHA ET AL: "Hyperspectral reconstruction from RGB images for vein visualization", PROCEEDINGS OF THE 11TH ACM MULTIMEDIA SYSTEMS CONFERENCE, ACMPUB27, NEW YORK, NY, USA, 27 May 2020 (2020-05-27), pages 77 - 87, XP058459537, ISBN: 978-1-4503-6845-2, DOI: 10.1145/3339825.3391861
- SEROUL PIERRE ET AL: "Hyper-spectral imaging system for in-vivo quantification of skin pigments", PROCEEDINGS OF THE 28TH IFSCC CONGRESS (27-10-2014 TO 30-10-2014), 30 October 2014 (2014-10-30), Paris, France, pages 123 - 132, XP093144955, Retrieved from the Internet <URL:https://hal.science/hal-01080593/file/Hyper-spectral%20imaging%20system%20for%20in-vivo%20skin%20pigments%20quantification.pdf> [retrieved on 20240325]
- HE QINGHUA ET AL: "Hyperspectral imaging enabled by an unmodified smartphone for analyzing skin morphological features and monitoring hemodynamics", BIOMEDICAL OPTICS EXPRESS, vol. 11, no. 2, 14 January 2020 (2020-01-14), United States, pages 895, XP055965074, ISSN: 2156-7085, DOI: 10.1364/BOE.378470
- PARK SANG MOK ET AL: "mHealth spectroscopy of blood hemoglobin with spectral super-resolution", vol. 7, no. 6, 1 June 2020 (2020-06-01), pages 563 - 573, XP055835687, Retrieved from the Internet <URL:https://www.osapublishing.org/DirectPDFAccess/9FA332C2-EAA5-4846-95ECBC5A0B796EBF_431957/optica-7-6-563.pdf?da=1&id=431957&seq=0&mobile=no> DOI: 10.1364/OPTICA.390409
- LI SHIWEI ET AL: "Quantitative Analysis of Skin using Diffuse Reflectance for Non-invasive Pigments Detection", 10 February 2021 (2021-02-10), pages 604 - 614, XP093144972, ISBN: 978-989-7584-88-6, Retrieved from the Internet <URL:https://www.scitepress.org/PublishedPapers/2021/103268/103268.pdf> [retrieved on 20240325], DOI: 10.5220/0010326806040614
- GEVAUX LOU ET AL: "Real-time skin chromophore estimation from hyperspectral images using a neural network", SKIN RESEARCH AND TECHNOLOGY, vol. 27, no. 2, 17 July 2020 (2020-07-17), DK, pages 163 - 177, XP093377313, ISSN: 0909-752X, DOI: 10.1111/srt.12927

## Description

### Technical Field

The disclosure relates to an electronic device. For example, the disclosure relates to a method and electronic device for determining skin pigmentation by an electronic device using hyper spectral reconstruction.

### Background Art

Generally, there is a high demand for skin pigmentation assessment to determine skin tone, ultraviolet exposure risk, pigmentation, psoriasis, eczema and any other skin abnormalities for applications such as cosmetics, dermatology, biometrics, and many others.

In conventional methods of skin pigmentation assessment of different tissues such as hair, skin, and blood is possible only after isolating individual tissue that is invasive. Thus there is a need for a skin pigmentation assessment method that can perform skin pigmentation assessment of different tissues present under the skin non-invasively that is without the need to isolate the individual tissue.

Furthermore, in various other conventional methods, skin pigmentation assessments are performed using specialized hyper spectral (HS) imaging systems with high resolution which are costly and inaccessible, Thus the image data acquired from consumer electronic devices such as a camera or a smart phone are limited by spectral resolution and are unsuitable for the conventional skin pigmentation assessments. As a result, there is a demand for a low-cost and easily accessible method of analyzing skin pigmentation.
Prior art includes: a publication to Sharma Neha et al. "Hyperspectral reconstruction from RGB images for vein visualization", in the Proceedings of the 11th ACM Multimedia Systems Conference, pages 77-87, in New Year, USA on 27 May 2020; a publication to Seroul Pierre et al: "Hyper-spectral imaging system for in-vivo quantification of skin pigments" in the Proceedings of the 28th IFSCC Congress, pages 123-132, on 30 October 2014, in Paris, France; a publication to Park Sang Mok et al: "mHealth spectroscopy of blood hemoglobin with spectral super-resolution", Optica, vol. 7, no.6, pages 563-573, on 1 June 2020; and a publication to Gevaux Lou et al. "Real-time skin chromophore estimation from hyperspectral images using a neural network", Skin Research and Technology, vol. 27, no. 2, pages 163-177 on 17 July 2020.

### Disclosure

### Technical Problem

Thus, it is desired to address the above mentioned disadvantages or other shortcomings or at least provide a useful alternative for skin pigmentation assessment.

### Technical Solution

The invention concerns an electronic device according to claim 1.

Optional features of the electronic device are provided in dependent claims 2 to 6.

These and other aspects of various example embodiments herein will be better appreciated and understood when considered in conjunction with the following description and the accompanying drawings. It should be understood, however, that the following descriptions, while indicating various example embodiments and numerous specific details thereof, are given by way of illustration and not of limitation.

### Description of Drawings

Various example embodiments of the disclosure are illustrated in the accompanying drawings, throughout which like reference letters indicate corresponding parts in the various figures. Further, the above and other aspects, features and advantages of certain embodiments of the present disclosure will be more apparent from the detailed description, taken in conjunction with the accompanying drawings, in which:
FIG. 1 is a diagram illustrating a Fitzpatrick scale for numerical classification of skin pigmentation, according to the prior art;
FIG. 2 is a diagram illustrating a conventional skin pigmentation assessment tool, according to the prior art;
FIGS. 3A and 3B are diagrams illustrating a tool to analyze individual tissues under the skin after shaving, according to the prior art;
FIG. 4 is a diagram illustrating a method which uses RGB data for skin pigmentation analysis, according to the prior art;
FIG. 5 is a block diagram illustrating an example configuration of an electronic device for determining skin pigmentation using hyper spectral reconstruction, according to various embodiments;
FIG. 6 is a flowchart illustrating an example method for determining skin pigmentation using hyper spectral reconstruction, according to various embodiments;
FIG. 7 is a system diagram illustrating an example method for determining skin pigmentation using hyper spectral reconstruction, according to various embodiments;
FIG. 8 is a diagram illustrating different tissues with different light reflection characteristics, according to various embodiments;
FIG. 9 is a diagram illustrating a spectral reflectance curve obtained from a combination of the reflectance curves of various tissues present under the skin, according to various embodiments;
FIG. 10 is a diagram illustrating a component analysis to extract spectra of individual tissues, according to various embodiments;
FIG. 11 is a system diagram illustrating an example method of evaluating skin hyperpigmentation progression, according to various embodiments;
FIG. 12 is a system diagram illustrating an example method of evaluating skin disorder and recommending products, according to various embodiments;
FIG. 13 is a diagram illustrating an example method of providing personalized accurate insights and recommendations, according to various embodiments;
FIG. 14 is a flowchart illustrating an example method of monitoring efficacy of skin treatment and cosmetics, according to various embodiments;
FIG. 15 is a diagram illustrating a comparison of RGB image and hyper spectral image, according to various embodiments;
FIG. 16 is a diagram illustrating a "MAKE-UP" camera feature, according to various embodiments; and
FIG. 17 is a diagram illustrating an enhanced camera experience for users, according to various embodiments.

### Mode for the Invention

The various example embodiments herein and the various features and advantageous details thereof are explained more fully with reference to the non-limiting embodiments that are illustrated in the accompanying drawings and detailed in the following description. Descriptions of well-known components and processing techniques may be omitted so as to not unnecessarily obscure the embodiments herein. The various embodiments described herein are not necessarily mutually exclusive, as various embodiments can be combined with one or more other embodiments to form new embodiments. The term "or" as used herein, refers to a non-exclusive or, unless otherwise indicated. The examples used herein are intended merely to facilitate an understanding of ways in which the embodiments herein can be practiced and to further enable those skilled in the art to practice the embodiments herein. Accordingly, the examples should not be constructed as limiting the scope of the disclosure herein.

Various embodiments may be described and illustrated in terms of blocks which carry out a described function or functions. These blocks, which may be referred to herein as managers, units, modules, hardware components or the like, are physically implemented by analog and/or digital circuits such as logic gates, integrated circuits, microprocessors, microcontrollers, memory circuits, passive electronic components, active electronic components, optical components, hardwired circuits and the like, and may optionally be driven by firmware. The circuits may, for example, be embodied in one or more semiconductor chips, or on substrate supports such as printed circuit boards and the like. The circuits of a block may be implemented by dedicated hardware, or by a processor (e.g., one or more programmed microprocessors and associated circuitry), or by a combination of dedicated hardware to perform some functions of the block and a processor to perform other functions of the block. Each block of the embodiments may be physically separated into two or more interacting and discrete blocks without departing from the scope of the disclosure. Likewise, the blocks of the embodiments may be physically combined into more complex blocks without departing from the scope of the disclosure.

The accompanying drawings are provided to aid in understanding various technical features and it should be understood that the embodiments presented herein are not limited by the accompanying drawings. As such, the present disclosure should be construed to extend to any alterations, equivalents and substitutes in addition to those which are particularly set out in the accompanying drawings; the invention being defined by the appended claims.

Accordingly the embodiments herein disclose a method for determining skin pigmentation by an electronic device using hyper spectral reconstruction. The method further includes capturing a Red, Green, and Blue (RGB) image of a skin. The method further includes converting the RGB image into a hyper spectral image. The method further includes determining wavelength band by applying a wavelength reflectance model on the hyper spectral image and determining information of the skin by applying a neural network model on the wavelength bands.

Accordingly the embodiments herein disclose an electronic device for determining skin pigmentation using hyper spectral reconstruction, an electronic device includes a memory, a processor and a skin details detector, operably coupled to the memory and the processor. The skin details detector is configured to capture the RGB image of the skin. Further, the skin details detector is configured to convert the RGB image into the hyper spectral image. Further, the skin details detector is configured to determine at least one wavelength band by applying a wavelength reflectance model on the hyper spectral image and determine information of the skin by applying the neural network model on the wavelength bands.

In existing methods and systems, pigmentation analysis of different tissues such as hair, skin, and blood is possible only after isolating them invasively. Unlike existing methods and systems, the disclosed method enables accurate pigmentation analysis of different tissues present under the skin non-invasively, which is without the need to isolate the individual tissue.

In existing methods and systems, pigmentation analysis is performed using specialized HS imaging systems with high resolution (<1nm). Unlike existing methods and systems, the disclosed method combines information from multiple pixels of an image which allows it to operate with noisy and low resolution HS images (>10nm). Therefore, the disclosed method works well even with HS images reconstructed from RGB images obtained from cameras present in consumer electronics.

Unlike existing methods and systems, the disclosed method reconstructs hyperspectral image from RGB image. Further, the disclosed method uses reconstructed hyperspectral image of skin to analyze pigmentation of different tissues present under the skin.

Unlike existing methods and systems, the disclosed method deals with noisy and low resolution nature of reconstructed hyperspectral images for analysis. The disclosed model can provide accurate pigmentation analysis even with low resolution hyperspectral images (>10nm).

In existing methods and systems, a RGB data based model is used for pigmentation analysis. Unlike existing methods and systems, 18% performance improvement on test dataset in skin tone classification when using disclosed wavelength reflectance model over existing SOTA RGB image model.

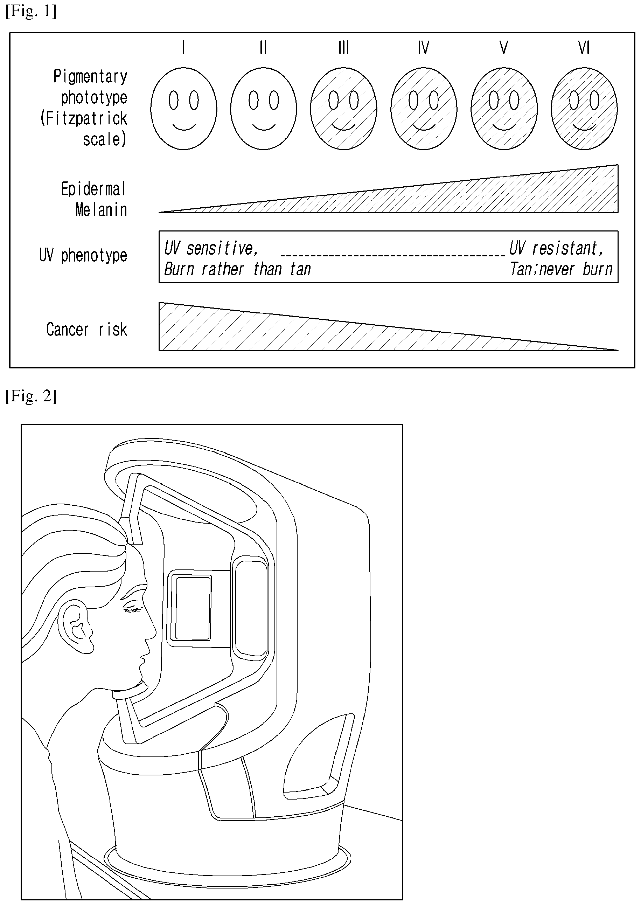

FIG. 1 is a diagram illustrating a Fitzpatrick scale for numerical classification of skin pigmentation, according to the prior art.

The Fitzpatrick scale is important to determine at least one of correct dose of Ultra Violet A (UVA) therapy, assess risk of sunburn, assess risk of skin cancer and cosmetics such as sunscreen.

In existing systems, the Fitzpatrick scale for skin type is determined by asking patients a set of questions. However, it is subjective and prone to inaccurate reporting and biases.

FIG. 2 is a diagram illustrating a conventional skin pigmentation assessment tool, according to the prior art.

FIG. 2 discloses one of an existing skin pigmentation assessment, the skin pigmentation assessment is performed using expensive and inaccessible Hyper Spectral (HS) imaging. Hence there is a demand for a non-invasive, cheap and accessible method to analyze skin pigmentation in detail as it has applications in cosmetics, dermatology and biometrics.

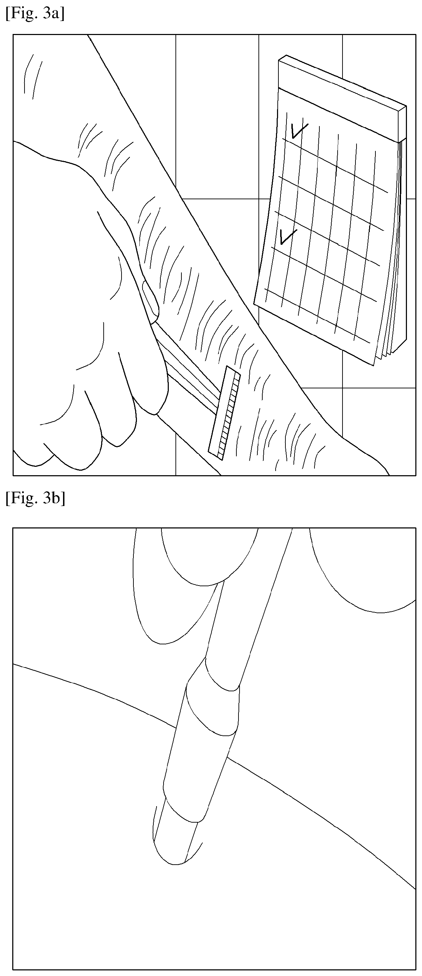

FIG. 3A and FIG. 3B are diagrams illustrating a tool to analyze individual tissues under the skin after shaving, according to the prior art.

FIG. 3A discloses a shaving requirement for the analysis.

FIG. 3B discloses the tool to analyze individual tissues under the skin after shaving.

Some existing system, requires shaving or biopsy to analyze individual tissues under the skin.

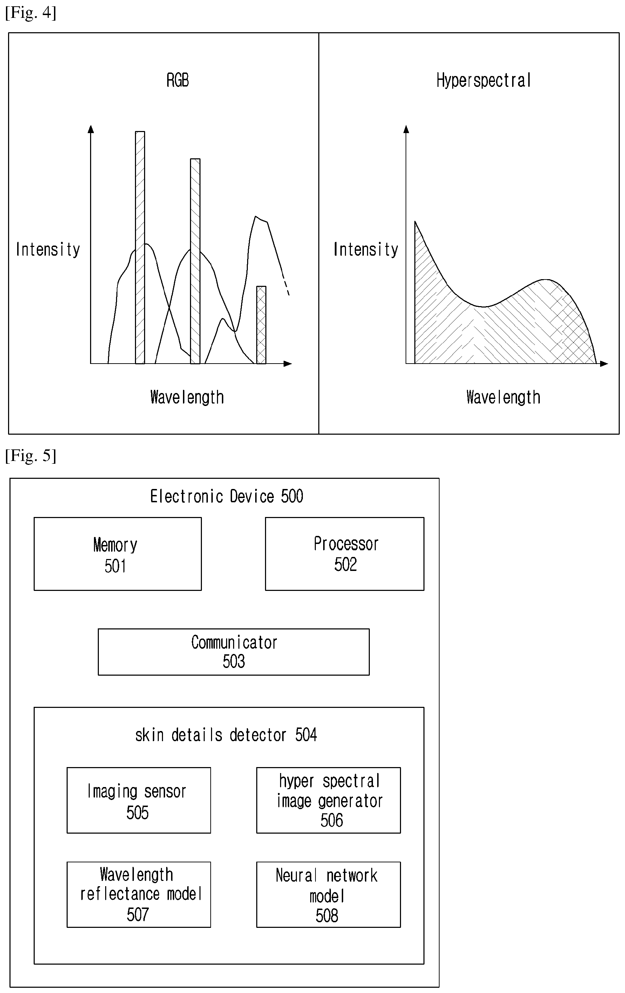

FIG. 4 is a diagram illustrating a method which uses RGB data for skin pigmentation analysis, according to the prior art.

Some other existing systems, uses a Red, Green, and Blue (RGB) data for skin pigmentation analysis, however the RGB data are limited by spectral resolution hence unfit for skin pigmentation analysis.

As there are narrow spectral bands (>30) in visible range, RGB images have only 3 bands in visible range. Further, RGB images have wide spectral bands and hence coarse pixel information. The disclosed method uses hyperspectral image which have narrow spectral bands resulting in detailed pixel information in an electromagnetic spectrum.

Referring now to the drawings and more particularly to FIGS. 5 through 17, where similar reference characters denote corresponding features consistently throughout the figures, these are shown various example embodiments.

FIG. 5 is a block diagram illustrating an example configuration of an electronic device for determining skin pigmentation using hyper spectral reconstruction, according to various embodiments.

Referring to FIG. 5, examples of the electronic device (500) include, but are not limited to a laptop, a palmtop, a desktop, a mobile phone, a smartphone, Personal Digital Assistant (PDA), a tablet, a wearable device, an Internet of Things (IoT) device, a virtual reality device, a foldable device, a flexible device, an immersive system, etc.

In an embodiment, the electronic device (500) includes a memory (501), a processor (e.g., including processing circuitry) (502), a communicator (e.g., including communication circuitry) (503), and a skin details detector (e.g., including various processing circuitry and/or executable program instructions) (504).

The memory (501) stores instructions to be executed by the processor (502). The memory (501) may include non-volatile storage elements. Examples of such non-volatile storage elements may include magnetic hard discs, optical discs, floppy discs, flash memories, or forms of electrically programmable memories (EPROM) or electrically erasable and programmable (EEPROM) memories. In addition, the memory (501) may, in some examples, be considered a non-transitory storage medium. The term "non-transitory" may indicate that the storage medium is not embodied in a carrier wave or a propagated signal. However, the term "non-transitory" should not be interpreted that the memory (501) is non-movable. In some examples, the memory (501) can be configured to store larger amounts of information than its storage space. In certain examples, a non-transitory storage medium may store data that can, over time, change (e.g., in Random Access Memory (RAM) or cache). The memory (501) can be an internal storage unit or it can be an external storage unit of the electronic device (500), a cloud storage, or any other type of external storage.

The processor (502) may include various processing circuitry and is configured to execute instructions stored in the memory (501). The processor (502) may be a general-purpose processor, such as a Central Processing Unit (CPU), an Application Processor (AP), or the like, a graphics-only processing unit such as a Graphics Processing Unit (GPU), a Visual Processing Unit (VPU) and the like. The processor (502) may include multiple cores to execute the instructions.

The communicator (503) may include various communication circuitry and is configured for communicating internally between hardware components in other user equipment or server. Further, the communicator (503) is configured to facilitate the communication between the electronic device (500) and other devices via one or more networks (e.g. Radio technology). The communicator (503) includes an electronic circuit specific to a standard that enables wired or wireless communication.

The skin details detector (504) may be implemented by processing circuitry such as logic gates, integrated circuits, microprocessors, microcontrollers, memory circuits, passive electronic components, active electronic components, optical components, hardwired circuits, or the like, and may optionally be driven by a firmware. The circuits may, for example, be embodied in one or more semiconductor chips, or on substrate supports such as printed circuit boards and the like.

The skin details detector (504) for determining skin pigmentation using hyper spectral reconstruction includes an imaging sensor (505), a hyper spectral image generator (506), a wavelength reflectance model (507) and a neural network model (508). The imaging sensor (505) captures a Red, Green, and Blue (RGB) image of a skin. The hyper spectral image generator (506) generates a hyper spectral image from the RGB image. The electronic device (500) determines at least one wavelength band by applying a wavelength reflectance model (507) on the hyper spectral image and determine information of the skin by applying a neural network model (508) on the wavelength bands.

The skin details detector (504) is configured to determine the wavelength band by applying the wavelength reflectance model on the hyper spectral image comprises segment different tissues under the skin non-invasively from the hyper spectral image using the wavelength reflectance model. The skin details detector (504) further configured to extract spectra of each individual tissue of the different tissues under the skin by analyzing multiple pixels on the hyper spectral image using the wavelength reflectance model and determine the at least one wavelength band comprising a concentration of pigments in the different tissues based on the extracted spectra of the individual tissues.

In an embodiment, the concentration of the pigments in the different tissues under the skin comprises information related to at least one of a thickness of the skin, a Melanin concentration in the skin, a Bilirubin concentration, a hair thickness under the skin, a Blood vessel thickness under the skin, a hemoglobin (Hb) concentration under the skin, and a oxygenated hemoglobin (HbO₂) concentration under the skin.

In an embodiment, the skin details detector (504) is configured to determine pigmentation information of the skin by applying the neural network model on the wavelength bands comprises input concentration of the concentration of pigments in the different tissues under the skin to the neural network model and obtain the pigmentation information of the skin from the neural network model.

The information of the skin includes at least one of a skin tone, an ultraviolet exposure risk, a pigmentation, a psoriasis, an eczema and a skin abnormalities.

In an embodiment, the RGB image is captured by at least one of an imaging apparatus with limited spectral resolution.

In an embodiment, the skin details detector (504) is configured to generate a pigmentation report by applying a wavelength reflectance model on the hyper spectral image and determining information of the skin by applying a neural network model on the wavelength bands. The skin details detector (504) further configured to determine whether the pigmentation is improving or in optimal range based on the pigmentation report. The skin details detector (504) further configured to perform recommend to the user not to change the prescription in response to determining that the pigmentation is improving or in optimal range or recommend to the user to change the prescription in response to determining that the extent of pigmentation is not improving or recommend to the user to stop medication and consult a doctor in response to determining that the extent of pigmentation is declining.

In an embodiment, the skin details detector (504) is configured to generate a skin health and disorder report after getting information from the neural network model. The neural network model uses the wavelength bands determined by the wavelength reflectance model. The skin details detector (504) is configured to display changes in health of the skin based on the skin health and disorders report or recommend products specific to the skin based on the skin health and disorder report.

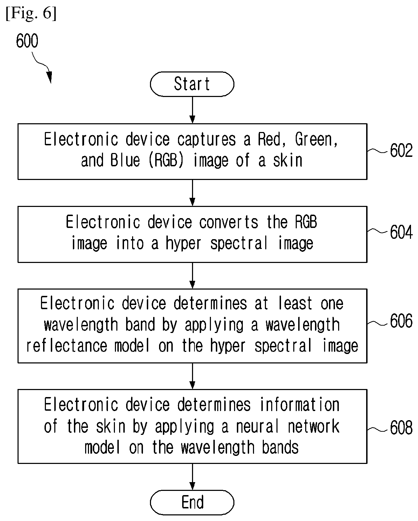

FIG. 6 is a flowchart illustrating an example method for determining skin pigmentation using hyper spectral reconstruction, according to various embodiments.

In the flowchart (600), at 602, the electronic device (500) captures a Red, Green, and Blue (RGB) image of a skin.

At 604, the electronic device (500) converts the RGB image into a hyper spectral image.

At 606, the electronic device (500) determines at least one wavelength band by applying a wavelength reflectance model (507) on the hyper spectral image.

At 608, the electronic device (500) determines information of the skin by applying a neural network model (508) on the wavelength bands.

The various actions, acts, blocks, steps, or the like in the method may be performed in the order presented, in a different order or simultaneously. Further, in various embodiments, some of the actions, acts, blocks, steps, or the like may be omitted, added, modified, skipped, or the like without departing from the scope of the disclosure.

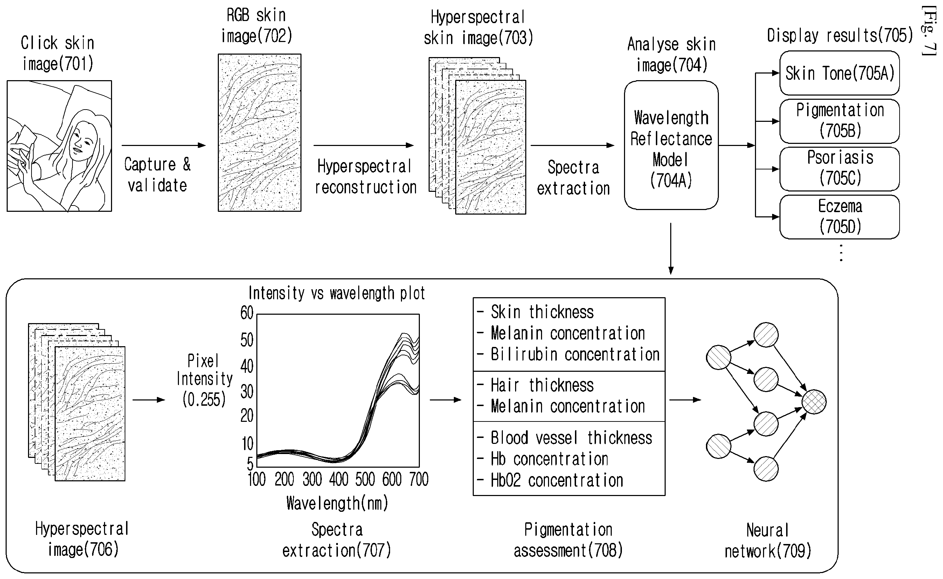

FIG. 7 is a system diagram illustrating an example method for determining skin pigmentation using hyper spectral reconstruction, according to various embodiments.

Referring to FIG. 7, an end to end system diagram illustrating a non-invasive and accessible method to analyze skin pigmentation using low resolution hyperspectral images of skin reconstructed from RGB camera images is shown. Further, the method may classify skin tone, predict UV exposure risk and skin disorders.

At 701, the electronic device (500) captures a Red, Green, and Blue (RGB) image of a skin.

At step, the electronic device (500) converts the RGB image into a hyper spectral image (703).

At step, the electronic device (500) determines at least one wavelength band by applying a wavelength reflectance model (704A) on the hyper spectral image at 706, wherein wavelength band information is a spectra as shown in 707.

At 708, skin thickness, melanin concentration, bilirubin concentration, hair thickness, melanin concentration, blood vessel thickness, hemoglobin (Hb) concentration, oxygenated hemoglobin (HbO₂) concentration, and pigmentation assessment details are extracted from the spectra.

At 705, the electronic device (500) determines information of the skin by applying a neural network model (709) on skin thickness, melanin concentration, bilirubin concentration, hair thickness, melanin concentration, blood vessel thickness, hemoglobin (Hb) concentration, oxygenated hemoglobin (HbO₂) concentration, to display results including skin tone (705A), pigmentation (705B), psoriasis (705C), eczema (705D).

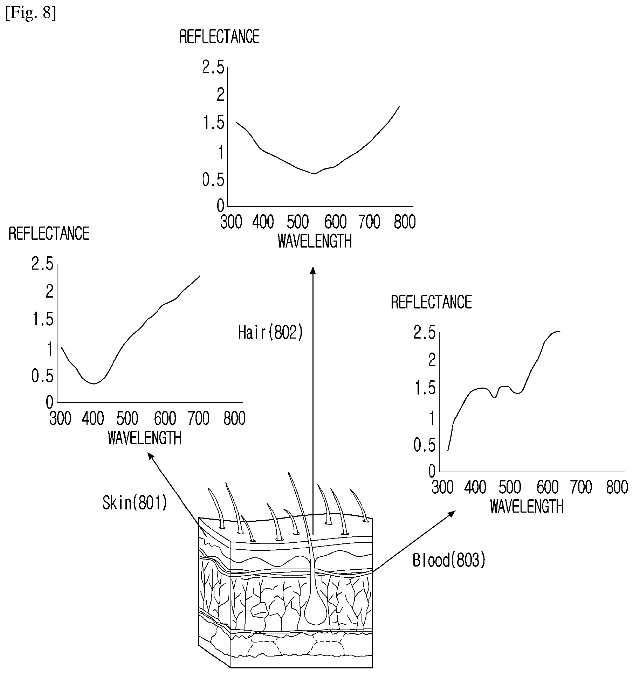

FIG. 8 is a diagram illustrating different tissues with different light reflection characteristics, according to various embodiments.

FIG. 8 illustrates different tissues such as skin (801), hair (802) and blood (803) having different light reflection characteristics.

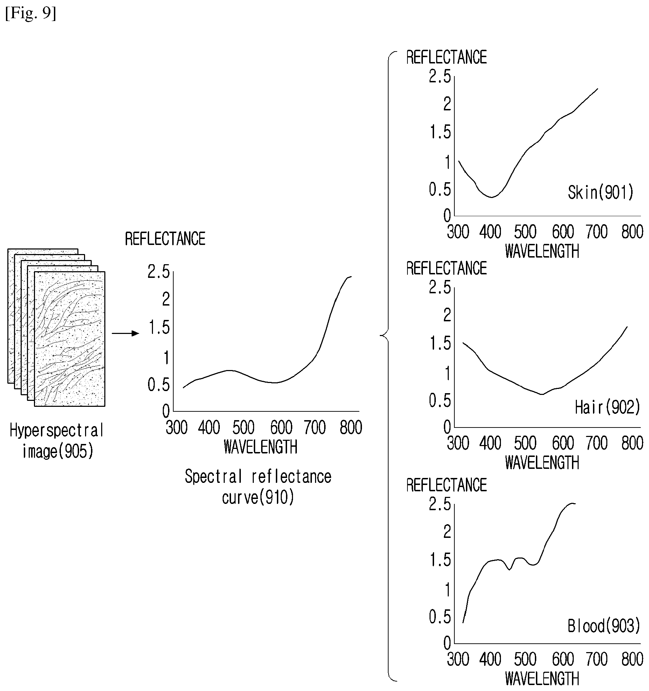

FIG. 9 is a diagram illustrating a spectral reflectance curve obtained from a combination of the reflectance curves of all the tissues present under the skin, according to various embodiments.

Referring to FIG. 9, when an image of skin is captured, it is converted into hyper spectral image (905), spectral reflectance curve (910) obtained from any pixel of that image is a combination of the reflectance curves of all the tissues present under the skin. To analyze pigmentation of a particular tissue, some examples are blood (903), hair (902), skin (901) etc., spectra of that tissue needs to be isolated. Unlike in conventional system, the analysis is possible only by invasive methods which involve isolating the tissue from the body and then analyzing its spectra.

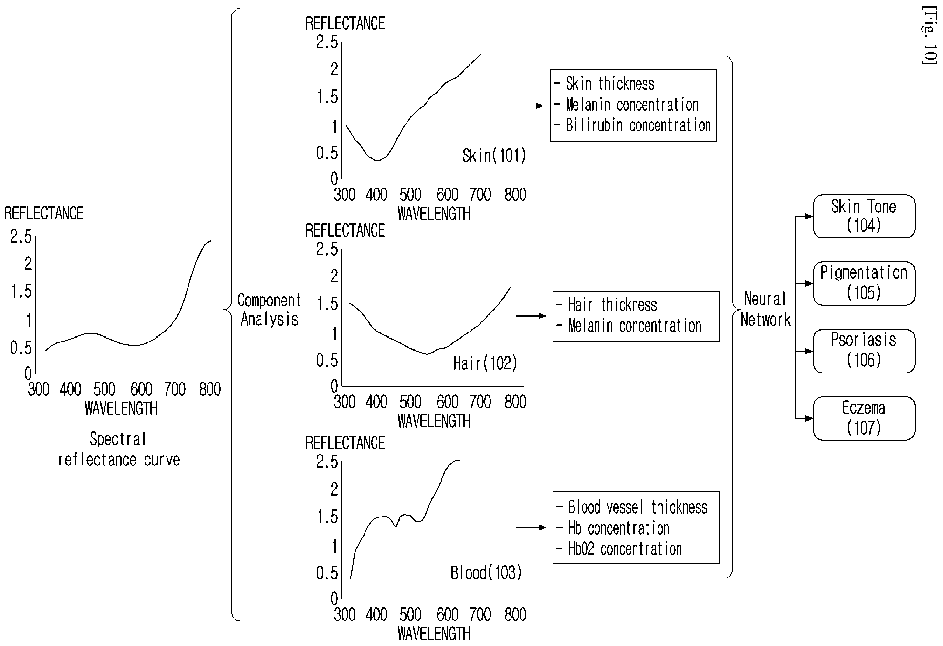

FIG. 10 is a diagram illustrating a component analysis to extract spectra of individual tissues, according to various embodiments.

Referring to FIG. 10, the disclosed wavelength reflectance model analyses multiple pixels on a skin image and uses component analysis to extract spectra of individual tissues. The spectra of individual tissues is investigated using Beer-Lambert law to find concentration of pigments. The Beer Lambert law equation is Absorbance = Molar coefficient * optical path length * concentration. Since there are multiple pigments in a tissue, Beer Lambert law gives a system of equations which can be solved only if there are multiple wavelengths at which reflection data is recorded such as skin (101), hair (102) and blood (103).

The wavelength of skin (101) reflects skin thickness, melanin concentration and bilirubin concentration.

The wavelength of hair (102) reflects hair thickness, melanin concentration.

The wavelength of blood (103) reflects blood vessel thickness, Hb concentration, HbO₂ concentration.

The neural network analyses and displays the skin Tone (104), pigmentation (105), psoriasis (106) and eczema (107).

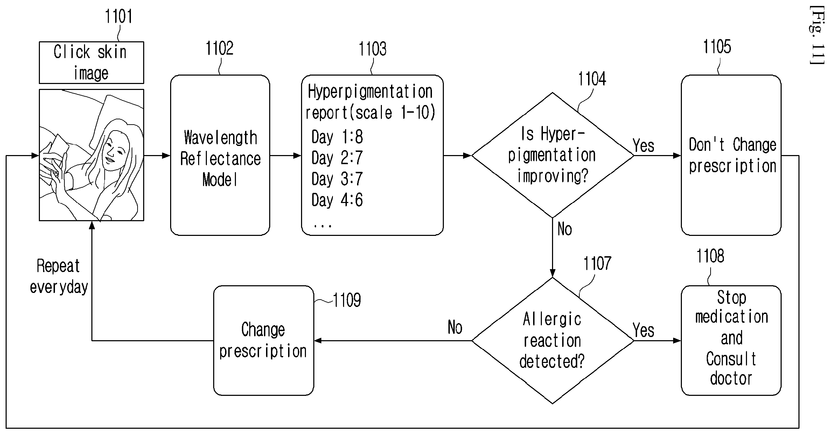

FIG. 11 a system diagram illustrating an example method of evaluating skin hyperpigmentation progression, according to various embodiments.

Referring to FIG. 11, an end to end system diagram illustrating an evaluation of skin hyperpigmentation progression at home and monitor treatment efficacy is provided.

In 1101, user captures a skin image, and it is analyzed by wavelength reflectance model (1102) to determine wavelength bands and provide a report of hyperpigmentation scale (1103).

In 1104, if the extent of pigmentation is improving, the electronic device recommends not to change prescription (1105). If the extent of pigmentation is not improving, the device checks for allergic reaction at 1107.

At 1108, the electronic device recommends to stop medication and to consult a doctor if any allergic reaction is detected in step 1107.

At 1109, the electronic device recommends to change the prescription if no allergic reaction is detected. The user can daily check the skin condition regularly using the electronic device.

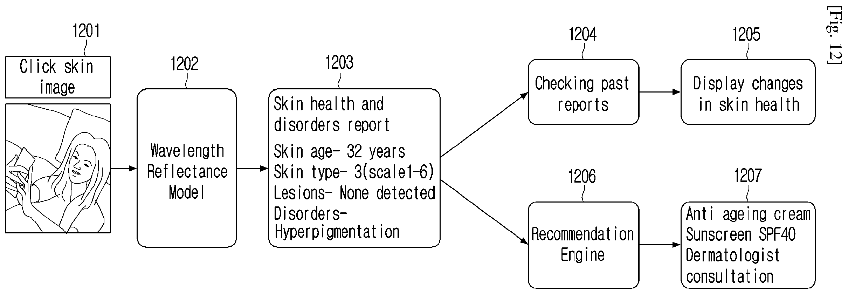

FIG. 12 is a system diagram illustrating an example method of evaluating skin disorder and recommending products, according to various.

Referring to FIG. 12, an end to end system diagram illustrating the Evaluation of skin for overall health and disorders and recommend personalized products from business partners is provided.

In an embodiment, in 1201, user captures a skin image, and it is analyzed by wavelength reflectance model (1202) to the determine wavelength bands.

In 1204, the electronic device checks the past reports and display daily changes in the skin health at 1205.

In 1205, a recommendation engine in the electronic device uses the generated hyperpigmentation report (1203) and at 1207, it recommends anti-ageing cream, sunscreen and dermatologist consultation.

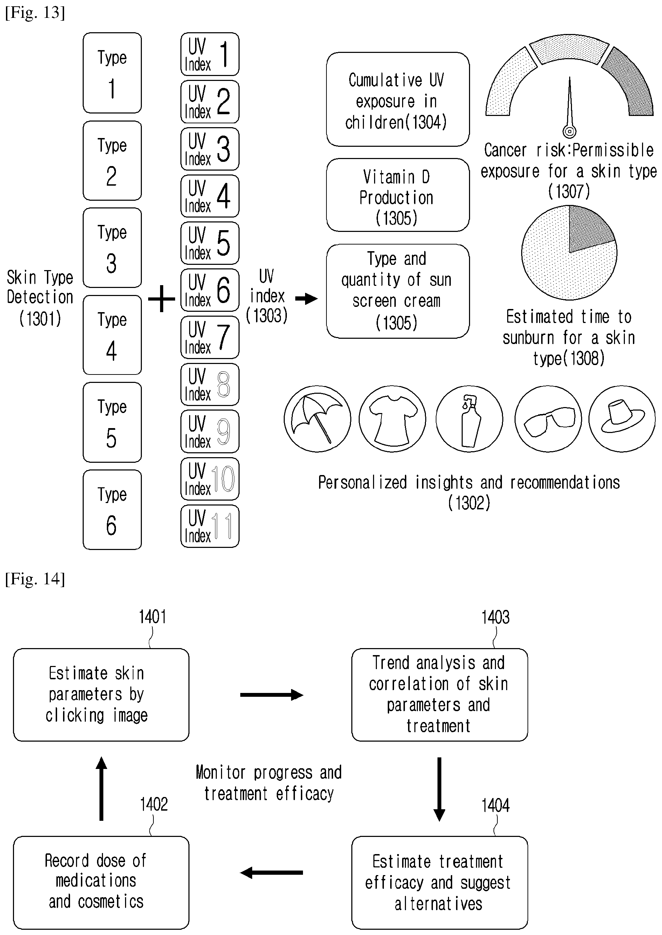

FIG. 13 is a diagram illustrating an example method of providing a personalized accurate insights and recommendation, according to various embodiments.

Referring to FIG. 13, a personalized and accurate insights related to UV exposure can be provided only after estimating melanin as it offers protection against skin cancers and sunburn due to Ultra Violet (UV).

The skin type detection (1301) checks the detected skin against the UV index to generate personalized accurate insights and recommendation (1302) such as Cumulative UV exposure in children (1304), vitamin D production (1305), type and quantity of sun screen cream (1306).

Further, the electronic device displays cancer risk monitor with permissible exposure for a skin type (1307). It may also display the estimated time to sunburn for a skin type (1308).

FIG. 14 is a flowchart illustrating an example method of monitoring efficacy of skin treatment and cosmetics, according to various embodiments.

Referring to FIG. 14 the flowchart illustrates an example of monitoring efficacy of skin treatment and cosmetics such as skin lightening products, treatment of dermatitis, hyperpigmentation and hypopigmentation medication.

At 1401, the electronic device may estimate skin parameters by clicking image.

At 1403, the electronic device may analyze the trend and correlation of skin parameters and treatment.

At 1404, the electronic device may estimate treatment efficacy and suggest alternatives.

At 1402, the electronic device may record dose of medications and cosmetics. The electronic device repeats the process.

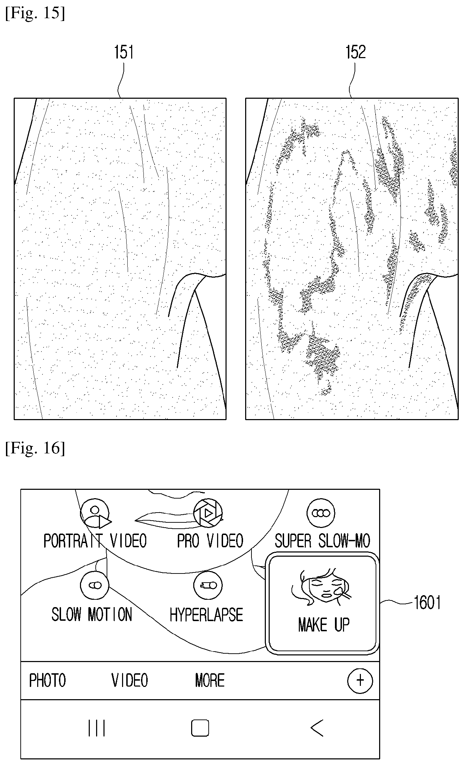

FIG. 15 is a diagram illustrating a comparison of RGB image and hyperspectral image, according to various embodiments.

Referring to FIG. 15, skin tone, health and abnormalities like sunburn, psoriasis, eczema etc. can be detected using hyperspectral image (152). Further RGB image (151) is not sufficient to analyze melanin and other pigments.

Deposition of melanin and other pigments can be estimated from skin image by analyzing reflectance at wavelengths between 400 - 700 nm from the hyperspectral image (152). The hyperspectral image (152) can provide objective and accurate estimate of skin tone and disorders.

FIG. 16 is a diagram illustrating a "MAKE-UP" (1601) camera feature, according to various embodiments.

Referring to FIG. 16, the "MAKE-UP" camera feature (1601) for pharma and companies can be utilized for features like Tone and undertone estimation of skin and lips wherein robust to lighting conditions. Further, the camera feature can also be utilized for dark circles and hyper-pigmented spots analysis. Further, the camera feature can also be utilized to recommend personalized products such as lotion, soap and sun screen. Further, the camera feature can also be utilized to help in buying right tone of foundation, nail paints and lipsticks with camera image effects.

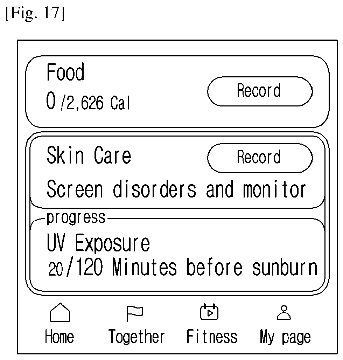

FIG. 17 is a diagram illustrating an enhanced camera experience for users, according to various embodiments.

Referring to FIG. 17, the enhanced camera experience to the user similar to, for example, the "Skin Care" feature in a Samsung health app. Further, the enhanced camera experience includes determination of pool user skin tone with weather data and UV index to warn about UV exposure and skin cancer risk. Further, the enhanced camera experience includes determination of screen common disorders such as hyper/hypo-pigmentation, bruises, scars, spider veins etc. Further, the enhanced camera experience includes determination of monitor skin attributes over time, identify trends and efficacy of cosmetic products.

While the disclosure has been illustrated and described with reference to various example embodiments, it will be understood that the various example embodiments are intended to be illustrative, not limiting. It will also be understood by those skilled in the art that various changes in form and detail may be made without departing from the full scope of the disclosure. It will also be understood that any of the embodiment(s) described herein may be used in conjunction with any other embodiment(s) described herein. The invention is defined by the appended claims.

## Claims

1. An electronic device (500) configured to determine information of skin using hyper spectral reconstruction, the electronic device comprising:
a memory (501);
a processor (502); and
a skin details detector (504) comprising circuitry, operably coupled to the memory and the processor, configured to:
capture a Red, Green, and Blue (RGB) image (151) of a skin;
convert the RGB image into a hyper spectral image (505);
determine at least one wavelength band by applying a wavelength reflectance model (507) on the hyper spectral image (152),
wherein determining the at least one wavelength band by applying the wavelength reflectance model on the hyper spectral image comprises:
segmenting different tissues under the skin non-invasively from the hyper spectral image using the wavelength reflectance model;
extracting spectra of each individual tissue of the different tissues under the skin by analysing multiple pixels on the hyper spectral image using the wavelength reflectance model; and
determining the at least one wavelength band based on the extracted spectra of the individual tissues; and
determine information of the skin by applying a neural network model (508) on the at least one wavelength band,
wherein the information of the skin includes at least one of a skin tone, an ultraviolet exposure risk, a pigmentation, a psoriasis and an eczema.

2. The electronic device as claimed in claim 1, wherein the at least one wavelength band includes a concentration of pigments in the different tissues based on the extracted spectra of the individual tissues.

3. The electronic device as claimed in claim 2, wherein the concentration of the pigments in the different tissues under the skin comprises information related to at least one of a thickness of the skin, a Melanin concentration in the skin, a Bilirubin concentration, a hair thickness under the skin, a Blood vessel thickness under the skin, a hemoglobin (Hb) concentration under the skin, and a oxygenated hemoglobin (HbO₂) concentration under the skin, wherein the information relates to a wavelength.

4. The electronic device as claimed in claim 2, wherein determining information of the skin by applying the neural network model on the at least one wavelength band comprises:
inputting concentration of the concentration of pigments in the different tissues under the skin to the neural network model; and
obtaining the information of the skin from the neural network model.

5. The electronic device as claimed in claim 1, wherein the electronic device is a consumer electronics device.

6. The electronic device as claimed in claim 1, wherein the electronic device is configured to:
generate a hyper pigmentation report by applying the wavelength reflectance model and neural network model containing information of an extent of pigmentation; and
determine whether the extent of pigmentation is improving or in optimal range based on the hyper pigmentation report; and
perform at least one of:
recommend not changing the prescription in response to determining that the extent of pigmentation is improving or in optimal range;
recommend changing the prescription in response to determining that the extent of pigmentation is not improving; and
recommend stopping medication and consulting a doctor in response to determining that the extent of pigmentation is declining.

## Patentansprüche

1. Elektronische Vorrichtung (500), die dazu konfiguriert ist, Informationen von Haut mittels Hyperspektralrekonstruktion zu bestimmen, wobei die elektronische Vorrichtung Folgendes umfasst:
einen Speicher (501);
einen Prozessor (502); und
einen Hautdetaildetektor (504), umfassend Schaltung, der an den Speicher und den Prozessor wirkgekoppelt ist, der zu Folgendem konfiguriert ist:
Erfassen eines Rot-, Grün- und Blau(RGB-)Bildes (151) einer Haut;
Umwandeln des RGB-Bildes in ein Hyperspektralbild (505);
Bestimmen von zumindest einem Wellenlängenband durch Anwenden eines Wellenlängenreflexionsmodells (507) auf das Hyperspektralbild (152),
wobei das Bestimmen des zumindest einen Wellenlängenbands durch Anwenden des Wellenlängenreflexionsmodells auf das Hyperspektralbild Folgendes umfasst:
Segmentieren von verschiedenen Geweben unter der Haut nicht-invasiv aus dem Hyperspektralbild mittels des Wellenlängenreflexionsmodells;
Extrahieren von Spektren jedes individuellen Gewebes der verschiedenen Gewebe unter der Haut durch Analysieren von mehreren Pixeln auf dem Hyperspektralbild mittels des Wellenlängenreflexionsmodells; und
Bestimmen des zumindest einen Wellenlängenbands basierend auf den extrahierten Spektren der individuellen Gewebe; und
Bestimmen von Informationen der Haut durch Anwenden eines neuronalen Netzmodells (508) auf das zumindest eine Wellenlängenband,
wobei die Informationen der Haut zumindest eines von einem Hautton, einem Ultraviolettexpositionsrisiko, einer Pigmentierung, einer Psoriasis und einem Ekzem beinhalten.

2. Elektronische Vorrichtung nach Anspruch 1, wobei das zumindest eine Wellenlängenband eine Konzentration von Pigmenten in den verschiedenen Geweben basierend auf den extrahierten Spektren der individuellen Gewebe beinhaltet.

3. Elektronische Vorrichtung nach Anspruch 2, wobei die Konzentration der Pigmente in den verschiedenen Geweben unter der Haut Informationen in Bezug auf zumindest eines von einer Dicke der Haut, einer Melaninkonzentration in der Haut, einer Bilirubinkonzentration, einer Haardicke unter der Haut, einer Blutgefäßdicke unter der Haut, einer Hämoglobin(Hb-)Konzentration unter der Haut und einer sauerstoffhaltigen Hämoglobin(HbO₂-)Konzentration unter der Haut umfasst, wobei sich die Informationen auf eine Wellenlänge beziehen.

4. Elektronische Vorrichtung nach Anspruch 2, wobei das Bestimmen von Informationen der Haut durch Anwenden des neuronalen Netzmodells auf das zumindest eine Wellenlängenband Folgendes umfasst:
Eingeben von Konzentration der Konzentration von Pigmenten in den verschiedenen Geweben unter der Haut in das neuronale Netzmodell; und
Erhalten der Informationen der Haut von dem neuronalen Netzmodell.

5. Elektronische Vorrichtung nach Anspruch 1, wobei die elektronische Vorrichtung eine Unterhaltungselektronikvorrichtung ist.

6. Elektronische Vorrichtung nach Anspruch 1, wobei die elektronische Vorrichtung zu Folgendem konfiguriert ist:
Erzeugen eines Hyperpigmentierungsberichts durch Anwenden des Wellenlängenreflexionsmodells und des neuronalen Netzmodells enthaltend Informationen über ein Ausmaß an Pigmentierung; und
Bestimmen, ob sich das Ausmaß an Pigmentierung verbessert oder in optimaler Spanne ist, basierend auf dem Hyperpigmentierungsbericht; und
Durchführen von zumindest einem von:
Empfehlen, die Verschreibung nicht zu ändern, als Reaktion auf das Bestimmen, dass sich das Ausmaß an Pigmentierung verbessert oder in optimaler Spanne ist;
Empfehlen, die Verschreibung zu ändern, als Reaktion auf das Bestimmen, dass sich das Ausmaß an Pigmentierung nicht verbessert; und
Empfehlen, Medikation abzubrechen und einen Arzt zu konsultieren, als Reaktion auf das Bestimmen, dass das Ausmaß an Pigmentierung abnimmt.

## Revendications

1. Dispositif électronique (500) configuré pour déterminer des informations de peau à l'aide d'une reconstruction hyper spectrale, le dispositif électronique comprenant :
une mémoire (501) ;
un processeur (502) ; et
un détecteur de détails cutanés (504) comprenant des circuits, couplés de manière opérationnelle à la mémoire et au processeur, configurés pour :
capturer une image rouge, verte et bleue (RVB) (151) d'une peau ;
convertir l'image RVB en une image hyper spectrale (505) ;
déterminer au moins une bande de longueur d'onde en appliquant un modèle de réflectance de longueur d'onde (507) sur l'image hyper spectrale (152),
dans lequel la détermination de l'au moins une bande de longueur d'onde en appliquant le modèle de réflectance de longueur d'onde sur l'image hyper spectrale comprend :
la segmentation non invasive de différents tissus sous la peau à partir de l'image hyper spectrale à l'aide du modèle de réflectance de longueur d'onde ;
l'extraction de spectres de chaque tissu individuel des différents tissus sous la peau en analysant plusieurs pixels sur l'image hyper spectrale à l'aide du modèle de réflectance de longueur d'onde ; et
la détermination de l'au moins une bande de longueur d'onde sur la base des spectres extraits des tissus individuels ; et
déterminer des informations de la peau en appliquant un modèle de réseau neuronal (508) sur l'au moins une bande de longueur d'onde,
dans lequel les informations de la peau comprennent au moins un parmi un teint cutané, un risque d'exposition aux ultraviolets, une pigmentation, un psoriasis et un eczéma.

2. Dispositif électronique tel que revendiqué dans la revendication 1, dans lequel l'au moins une bande de longueur d'onde comprend une concentration de pigments dans les différents tissus sur la base des spectres extraits des tissus individuels.

3. Dispositif électronique tel que revendiqué dans la revendication 2, dans lequel la concentration des pigments dans les différents tissus sous la peau comprend des informations relatives à au moins une parmi une épaisseur de la peau, une concentration de mélanine dans la peau, une concentration de bilirubine, une épaisseur de poil sous la peau, une épaisseur de vaisseau sanguin sous la peau, une concentration d'hémoglobine (Hb) sous la peau et une concentration d'hémoglobine oxygénée (HbO₂) sous la peau, dans lequel les informations se rapportent à une longueur d'onde.

4. Dispositif électronique tel que revendiqué dans la revendication 2, dans lequel la détermination d'informations de la peau en appliquant le modèle de réseau neuronal sur l'au moins une bande de longueur d'onde comprend :
la soumission de la concentration de pigments dans les différents tissus sous la peau au modèle de réseau neuronal ; et
l'obtention des informations de la peau à partir du modèle de réseau neuronal.

5. Dispositif électronique tel que revendiqué dans la revendication 1, dans lequel le dispositif électronique est un dispositif électronique grand public.

6. Dispositif électronique tel que revendiqué dans la revendication 1, dans lequel le dispositif électronique est configuré pour :
générer un rapport d'hyperpigmentation en appliquant le modèle de réflectance de longueur d'onde et le modèle de réseau neuronal contenant des informations d'une étendue de pigmentation ; et
déterminer si l'étendue de pigmentation s'améliore ou se trouve dans une plage optimale sur la base du rapport d'hyperpigmentation ; et
réaliser au moins une parmi :
recommander de ne pas modifier la prescription en réponse à la détermination du fait que l'étendue de la pigmentation s'améliore ou se situe dans une plage optimale ;
recommander de modifier la prescription en réponse à la détermination du fait que l'étendue de la pigmentation ne s'améliore pas ; et
recommander l'arrêt de médicaments et la consultation d'un médecin en réponse à la détermination du fait que l'étendue de pigmentation diminue.
